(19) Europäisches Patentamt
European Patent Office
Office européen des brevets

(11) Veröffentlichungsnummer : **0 278 915 B1**

(12) # EUROPÄISCHE PATENTSCHRIFT

(45) Veröffentlichungstag der Patentschrift :
**27.11.91 Patentblatt 91/48**

(21) Anmeldenummer : **88810075.7**

(22) Anmeldetag : **08.02.88**

(51) Int. Cl.[5] : **C07D 317/46**, C07D 317/52, C07D 317/54, C07D 327/04, C07D 339/06, C07D 317/64, A01N 43/28, A01N 43/30, C07D 407/06, C07D 411/06

(54) **Diphenyläthylen-Derivate.**

(30) Priorität : **13.02.87 CH 533/87**
**14.01.88 CH 122/88**

(43) Veröffentlichungstag der Anmeldung :
**17.08.88 Patentblatt 88/33**

(45) Bekanntmachung des Hinweises auf die Patenterteilung :
**27.11.91 Patentblatt 91/48**

(84) Benannte Vertragsstaaten :
**AT BE CH DE FR GB IT LI NL**

(56) Entgegenhaltungen :
**EP-A- 0 163 537**
**GB-A- 2 176 785**
**US-A- 4 548 941**

(56) Entgegenhaltungen :
**JOURNAL OF ORGANIC CHEMISTRY, Band 39, Nr. 9, 1974, Seiten 1242-1247, Washington, US; D.T. WITIAK et al.: "Vilsmeier-Haack cyclizations. Synthesis of 2-substituted 3-dimethylamino-5,6-methylenedioxyindenes and the corresponding indanones"**
**JOURNAL OF ORGANIC CHEMISTRY, Band 50, 1985, Seiten 1087-1105, American Chemical Society, Washington, US; M.E. JUNG et al.: "Stereoselective synthesis of an analogue of podophyllotoxin by an intramolecular diels-alder reaction"**
**Bull. soc.chim.France 1969, 2787/8;**
**J.Heterocyclic Chem.21, 591(1984)**

(73) Patentinhaber : **CIBA-GEIGY AG**
**Klybeckstrasse 141**
**CH-4002 Basel (CH)**

(72) Erfinder : **Waespe, Hans-Rudolf, Dr.**
**Feldstrasse 86**
**CH-4123 Allschwil (CH)**

EP 0 278 915 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte α,α-Diphenyläthylen-Derivate, Verfahren zu ihrer Herstellung, ihre Zwischenprodukte und ihre Verwendung zur Bekämpfung von Schädlingen.

Die neuen erfindungsgemässen Verbindungen haben die allgemeine Formel I

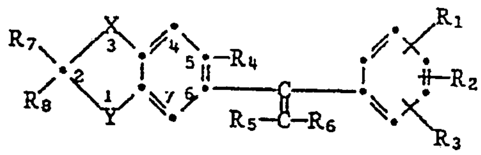

(I)

worin

- $R_1$ und $R_4$ unabhängig voneinander Wasserstoff, Hydroxy, $C_1$-$C_5$-Alkyl, $C_1$-$C_8$-Alkoxy, mit 1 bis 7 Halogenatomen substituiertes $C_1$-$C_3$-Alkoxy, Alkoxyalkoxy mit insgesamt 2 bis 6 C-Atomen, $C_3$-$C_5$-Alkenyloxy oder $C_3$-$C_5$-Alkinyloxy ;
- $R_2$ und $R_3$ unabhängig voneinander Wasserstoff, Halogen, $C_1$-$C_3$-Alkyl, $C_1$-$C_5$-Alkoxy oder Nitro ; oder
- $R_1$ und $R_2$ zusammen einen Rest —O-$CH_2$-O— oder —O-$CH_2$-$CH_2$-O— ;
- $R_5$ und $R_6$ unabhängig voneinander Wasserstoff, Halogen oder Methyl ;
- $R_7$ und $R_8$ unabhängig voneinander Wasserstoff, Methyl oder Aethyl ; und
- X und Y unabhängig voneinander —O— oder —S— bedeuten, mit der Maßgabe, daß mindestens einer der Substituenten $R_1$ bis $R_8$ eine andere Bedeutung als Wasserstoff hat, wenn X und Y beide für —O— stehen.

Bevorzugt werden erfindungsgemäss Verbindungen der Formel I, die dadurch gekennzeichnet sind, dass

- $R_1$ und $R_4$ unabhängig voneinander Wasserstoff, $C_1$-$C_8$-Alkoxy, mit 1 bis 7 Halogenatomen substituiertes $C_1$-$C_3$-Alkoxy, Alkoxyalkoxy mit insgesamt 2 bis 6 C-Atomen, $C_3$-$C_5$-Alkenyloxy oder $C_3$-$C_5$-Alkinyloxy ;
- $R_2$ und $R_3$ unabhängig voneinander Wasserstoff, Halogen, $C_1$-$C_3$-Alkyl, $C_1$-$C_5$-Alkoxy oder Nitro ; oder
- $R_1$ und $R_2$ zusammen einen Rest —O-$CH_2$-O— oder —O-$CH_2$-$CH_2$-O— ;
- $R_5$ und $R_6$ unabhängig voneinander Wasserstoff, Halogen oder Methyl ;
- $R_7$ und $R_8$ unabhängig voneinander Wasserstoff, Methyl oder Aethyl ;

X —O— oder —S— ; und Y —O— bedeuten, mit der Maßgabe ; daß mindestens einer der Substituenten $R_1$ bis $R_8$ eine andere Bedeutung als Wasserstoff hat, wenn X und Y beide für —O— stehen.

Von besonderer Bedeutung sind erfindungsgemäss die Verbindungen der Formeln Ia und Ib :

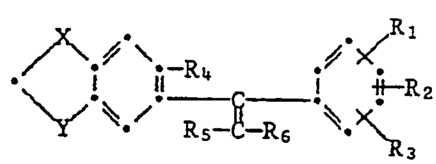

(Ia),

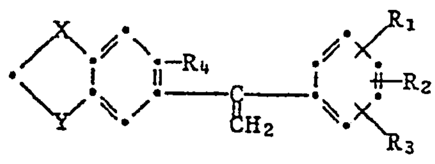

(Ib),

in welchen Formeln $R_1$ bis $R_6$, X und Y die oben angegebenen Bedeutungen haben.

Wegen ihrer vorteilhaften Wirkung werden solche Verbindungen der Formeln I, bzw. Ia und Ib bevorzugt, worin

- $R_1$ und $R_4$ unabhängig voneinander Wasserstoff, $C_1$-$C_5$-Alkoxy, mit 1 bis 5 Fluor- oder Chloratomen substituiertes $C_1$-$C_2$-Alkoxy, Alkoxyalkoxy mit insgesamt 2 bis 5 C-Atomen oder Propinyloxy ;

$R_2$ und $R_3$ unabhängig voneinander Wasserstoff, Fluor, Chlor, Methoxy oder Aethoxy ; oder
$R_1$ und $R_2$ zusammen den Rest —O-$CH_2$-O— oder —O-$CH_2$-$CH_2$-O— ; und
$R_5$ und $R_6$ unabhängig voneinander Wasserstoff, Halogen oder Methyl bedeuten, mit der Maßgabe, daß mindestens einer der Substituenten $R_1$ bis $R_8$ eine andere Bedeutung als Wasserstoff hat, wenn X und Y beide für —O— stehen ; sowie solche Verbindungen der Formel Ib, worin

$R_1$ Methoxy, Aethoxy oder Trifluormethoxy ;
$R_2$ und $R_3$ unabhängig voneinander Wasserstoff oder Fluor ; und
$R_4$ $C_1$-$C_8$-Alkoxy bedeuten.

Speziell hervorzuheben sind die erfindungsgemässen Verbindungen der Formel Ic

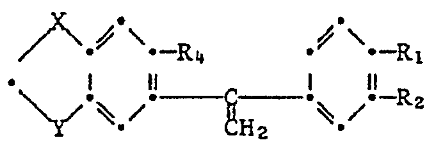

(Ic),

worin $R_1$, $R_2$ und $R_4$ sowie X und Y die oben angegebenen Bedeutungen haben. Von besonderer Bedeutung sind diejenigen erfindungsgemässen Verbindungen, bei denen X und Y gleichzeitig —O— bedeuten, wobei die vorstehend definierte Maßgabe gilt, diejenigen, bei denen —S— und Y —O— bedeuten, sowie diejenigen, bei denen $R_2$ Fluor bedeutet.

Unter dem Begriff Alkyl — auch als Bestandteil von Alkoxygruppen — sind im Rahmen der vorliegenden Erfindung geradkettige und verzweigte Alkylreste und je nach Zahl der angegebenen Kohlenstoffatome beispielsweise folgende Gruppen zu verstehen : Methyl, Aethyl, Propyl, Butyl, Pentyl usw. sowie ihre Isomeren, wie z.B. Isopropyl, Isobutyl, tert.-Butyl, Isopentyl, Neopentyl usw. Unter Halogen sind Fluor, Chlor, Brom, und Jod, vorzugsweise Fluor oder Chlor, zu verstehen.

Unter dem Begriff Halogenalkoxy im Rahmen der vorliegenden Erfindung sind geradkettige und verzweigte Reste, wie Methoxy, Aethoxy, n-Propoxy und Isopropoxy zu verstehen, die mit bis zu 7 unterschiedlichen oder gleichartigen Halogenatomen substituiert sind, wobei es sich um perhalogenierte Alkylreste oder auch um solche handeln kann, deren Wasserstoffatome nur teilweise durch Halogen, vorzugsweise Fluor oder Chlor, substituiert sind.

Die Verbindungen der Formel I können analog an sich bekannter Verfahren hergestellt werden [vgl. z.B. Fieser & Fieser, "Reagents for Organic Synthesis" Bd. 1, 572, Wiley (1967) ; Jones et al. Organic Reactions 6, 339 (1951) ; J. Am. Chem. Soc. 54, 1957 (1932) ; J. March, "Advanced Organic Chemistry", 836, McGraw-Hill, 2nd ed. (1977)]. So kann man erfindungsgemäss Verbindungen der Formel I — einschliesslich derjenigen der Formeln Ia, Ib und Ic — erhalten, indem man eine Verbindung der Formel II

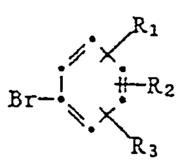

(II)

in einer Grignard-Reaktion in Gegenwart von Magnesium oder unter Einwirkung von Lithiumalkylen, z.B. Butyllithium, mit einer Verbindung der Formel III

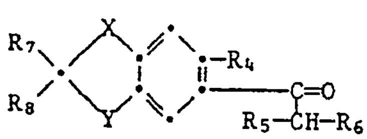

(III),

umsetzt, wobei in den Formeln II und III $R_1$ bis $R_8$ die vorstehend angegebenen Bedeutungen haben.

Verbindungen der Formel I, worin X und Y gleichzeitig für —O— stehen, können dadurch hergestellt werden, dass man eine Verbindung der Formel IV

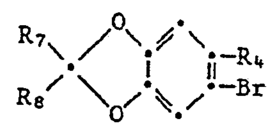

(IV)

in einer Grignard-Reaktion In Gegenwart von Magnesium oder in Gegenwart eines Lithiumalkyls, z.B. Butyllithium, mit einer Verbindung der Formel V

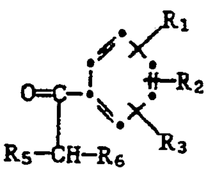

(V)

umsetzt, wobei in den Formeln IV und V $R_1$ bis $R_8$ die vorstehend angegebenen Bedeutungen haben.

Bei den oben aufgeführten Umsetzungen kann in einigen Fällen in Abhängigkeit von den Substituenten an den Phenylresten der Verbindungen der Formel II bis V ein entsprechendes Carbinol der Formel VI

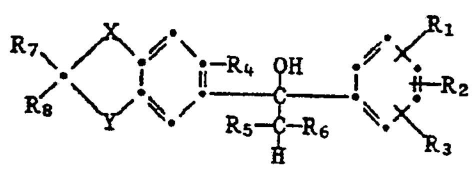

(VI)

(gegebenenfalls im Gemisch mit dem Endprodukt der Formel I) gebildet werden (vgl. J. Org. Chem. 29, 2527). Wenn sich solche Diphenylcarbinole der Formel VI isolieren lassen, können auch diese mit aciden Reagentien (z.B. Kaliumhydrogensulfat, p-Toluolsulfonsäure) in an sich bekannter Weise in eine Verbindung der Formel I übergeführt werden (vgl. Synthesis 1985, 1159).

Zur Herstellung von Verbindungen der Formel I, worin $R_5$ und $R_6$ Wasserstoff oder Halogen, z.B. Chlor, bedeuten, kann auch die Wittig-Reaktion, d.h. die Olefinierung der Carbonylfunktion in einer Verbindung der Formel VII herangezogen werden (vgl. J. Am. Chem. Soc. 82, 1260 ; Synth. Commun. 15 (10), 855), wie z.B.:

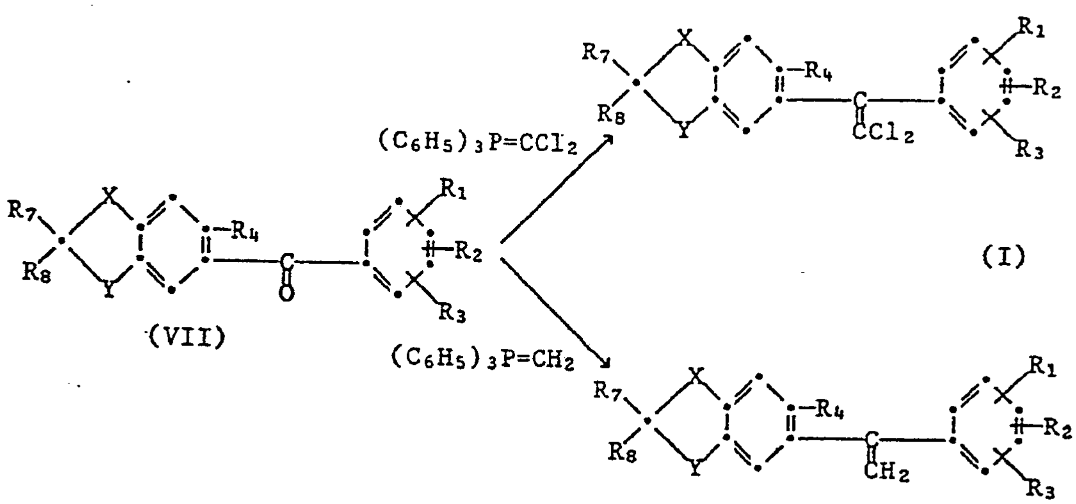


(VII)

(I)

In den obigen Formeln haben $R_1$ bis $R_8$, X und Y die vorhergehend angegebenen Bedeutungen.

Die Ausgangsstoffe der vorstehenden Formeln II, IV und V sind zumeist bekannt un können — falls sie neu sind — analog bekannter Verfahren hergestellt werden. Bei den Ausgangsverbindungen der Formel III handelt es sich teilweise um neuartige Substanzen, die dann ebenfalls einen Gegenstand der vorliegenden Erfindung bilden und in an sich bekannter Weise erhältlich sind.

Von den Verbindungen der Formel III sind diejenigen neu, in denen

R Wasserstoff, Hydroxy, $C_1$-$C_5$-Alkyl, $C_1$-$C_8$-Alkoxy, mit 1 bis 7 Halogenatomen substituiertes $C_1$-$C_3$ -Alkoxy, Alkoxyalkoxy mit insgesamt 2 bis 6 C-Atomen, $C_3$-$C_5$ -Alkenyloxy oder $C_3$-$C_5$ -Alkinyloxy,
$R_5$ und $R_6$ unabhängig voneinander Wasserstoff, Halogen oder Methyl,
$R_7$ und $R_8$ unabhängig voneinander Wasserstoff, Methyl oder Methyl und
X und Y unabhängig voneinander —O— oder —S— bedeuten, mit der Massgabe, dass in denjenigen Verbindungen der Formel III, in denen

i) $R_4$, $R_7$ und $R_8$ Wasserstoff und X und Y beide —O— bedeuten und einer der beiden Substituenten $R_5$ und $R_6$ für Wasserstoff steht, der andere dieser beiden Substituenten für Halogen steht ;
ii) $R_4$ Hydroxy oder Methoxy, $R_7$ und $R_8$ Wasserstoff und X und Y beide —O— bedeuten, mindestens einer der beiden Substituenten $R_5$ und $R_6$ für Halogen oder Methyl steht ;
iii) $R_4$ Wasserstoff und von den beiden Substituenten $R_5$ und $R_6$ der eine Wasserstoff und der andere Chlor bedeutet und X für —O— und Y für —S— steht, von den Substituenten $R_7$ und $R_8$ entweder mindestens einer für Wasserstoff oder einer für Methyl und der andere für Aethyl steht ;
iv) $R_4$, $R_5$ und $R_6$ Wasserstoff bedeuten und X für —O— und Y für —S— steht, von den beiden Substituenten $R_7$ und $R_8$ der eine Wasserstoff und der andere Methyl oder Aethyl bedeutet.

Man kann die substituierten Acetophenone der Formel III durch Acylierung nach Friedel-Crafts herstellen [vgl. z.B. Org. Synth., Coll. Vol. 3, 23 (1955) ; J. Chem. Soc. B, 1343 (1970) ; J. March, "Advanced Organic Chemistry" Mc Graw-Hill, 2. ed., 490 (1977)], indem man eine Verbindung der Formel VIII

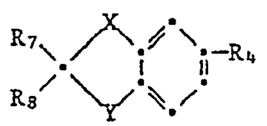

(VIII)

in Gegenwart eines Friedel-Crafts-Katalysators, d.h. einer Lewis-Säure, wie z.B. $AlCl_3$, mit einem Acylhalogenid der Formel IX

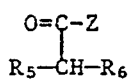

(IX)

umsetzt, wobei $R_4$ bis $R_8$, X und Y die oben angegebenen Bedeutungen haben und Z ein Halogenatom, vorzugsweise Chlor, bedeutet. In ähnlicher Weise sind auch die Benzophenon-Ausgangsprodukte der Formel VII durch übliche Friedel-Crafts-Acylierung zugänglich :

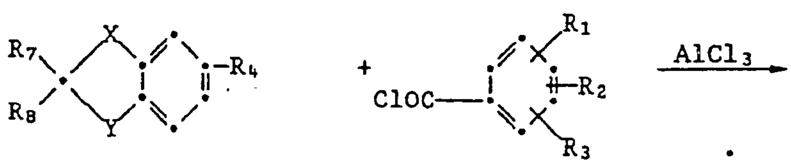


(VII)

In den obigen Formeln haben $R_1$ bis $R_8$, X und Y die vorhergehend angegebenen Bedeutungen.

Die 6-Brom-1,3-benzodioxole der Formel IV sind erhältlich durch Bromierung der entsprechenden 1,3-Benzodioxole [vgl. J. Am. Chem. Soc. 74, 1602 (1952) ; Austral. J. scient. Res. (A) 5, 206 (1952) ; J. Agr. Food Chem. 15 (1), 139 (1967)].

Verbindungen der Formel VIII, in denen $R_7$, $R_8$ für Wasserstoff und X für —S— steht, lassen sich herstellen, indem man vom 5-Hydroxy-1,3-benzoxathiol-2-on ausgeht, das wie folgt erhalten werden kann [vgl. J. Org. Chem. 33 (12), 4426-31 (1968)] :

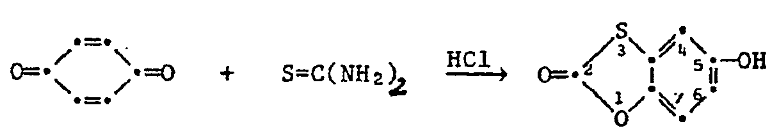


In diesen Verbindungen kann die 5-ständige Hydroxygruppe in üblicher Weise veräthert werden. 1,3-Benzoxathiol-2-one können mit Dibrommethan als Reagens für die phasentransferkatalysierte Ueberführung der 2-ständigen Carbonylgruppe zur Methylengruppe in entsprechende 1,3-Benzoxathiole der Formel VIII umgewandelt werden [vgl. Synth. Communic. 10 (2), 161 (1980)], wie z.B.

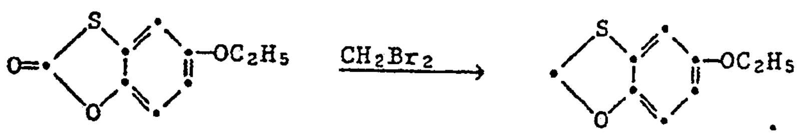


Das unsubstituierte alpha-Phenyl-alpha-(1,3-benzdioxol-5-yl)-äthylen ist aus J.Org.Chem. 39 (9), 1974, als Zwischenprodukt bei der Herstellung von Inden- und Indanon-Derivaten erwähnt, wobei jedoch keinerlei Angaben über irgendeine biologische Wirkung gemacht werden. Als weiteres Zwischenprodukt wird dort auch 3,4-Methylendioxy-acetophenon erwähnt, welches auch aus J.Org.Chem. 50 (7), 1985, bekannt ist. Ferner sind einige Derivate dieser letztgenannten Verbindung aus der europäischen Patentanmeldung 163537, der britischen Patentschrift 2176785, der amerikanischen Patentschrift 4548951, aus "Bulletin de la Société Chimique de France", Nr. 8, 1969, und aus J. Heterocyclic Chem. 21 (1984), bekannt.

Ueberraschenderweise wurde gefunden, dass die vorliegenden Verbindungen der Formel I (einschliesslich der Formeln Ia, Ib und Ic) bei guter Pflanzenverträglichkeit und geringer Fisch- und Warmblütertoxizität ausgezeichnete Wirksamkeit als Schädlingsbekämpfungsmittel aufweisen. Sie eignen sich vor allem zur Bekämpfung von Pflanzen und Tiere befallenden Insekten und Vertretern der Ordnung Akarina.

Insbesondere eignen sich die Verbindungen der Formel I zur Bekämpfung von Insekten der Ordnungen : Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien : Ixodidae, Argasidae, Tetranychidae und Dermanyssidae.

Neben ihrer Wirkung gegenüber Fliegen, wie z.B. Musca domestica, und Mückenlarven können die erfindungsgemässen Wirkstoffe auch zur Bekämpfung von pflanzenschädigenden Insekten in Zier- und Nutzpflanzungen, insbesondere in Baumwollkulturen (z.B. Spodoptera littoralis und Heliothis virescens), sowie in Obst- und Gemüsekulturen (z.B. Plutella xylostella, Laspeyresia pomonella, Leptinotarsa decemlineata und Epilachna varivestis) eingesetzt werden. Die Verbindungen der Formel I zeichnen sich durch eine ausgeprägte Wirksamkeit gegen pflanzenschädigende saugende Insekten und hohe larvizide Wirkung, insbesondere gegen Larven von fressenden Schadinsekten, aus. Werden die Wirkstoff-Verbindungen von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Wirkstoffe der Formel I können ferner zur Bekämpfung von Ektoparasiten, wie ektoparasitären Insekten, z.B. Lucilia sericata, sowie von Zecken, z.B. Boophilus microplus, an Haus- und Nutztieren eingesetzt werden, z.B. durch Tier-, Stall- und Weidebehandlung.

Bei der Behandlung von Weidetieren mit den erfindungsgemässen Wirkstoffen, z.B. mittels Viehbädern, Aufguss-Methoden oder Sprühgängen, wird durch die Adhäsionswirkung der Aktivsubstanzen ein lang anhaltender toxischer Effekt gegen Ektoparasiten, wie beispielsweise schädlichen Dipteren, auf der Haut und dem Fell der Tiere erzielt. Ein frühzeitiges Aus- oder Abwaschen der auf die Oberfläche der Nutztiere applizierten Wirkstoffe durch ablaufendes Regenwasser kann somit verhindert werden.

Die gute pestizide Wirkung der erfindungsgemässen Verbindungen der Formel I entspricht einer Abtötungsrate (Mortalität) von mindestens 50-60% der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht : Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die erfindungsgemässen Verbindungen werden in unveränderter Form oder vorzugsweise zusammen mit den in der Formulierungstechnik üblichen Hilfsmitteln eingesetzt und können daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieses Wirkstoffes mit anderen Insektiziden oder Akariziden, und gegebenenfalls einen festen oder flüssigen Zusatzstoff enthaltenden Mittel,

Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten.

Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual"
MC Publishing Corp., Ridgewood, New Jersey, 1979 ;

Dr. Helmut Stache "Tensid Taschenbuch", Carl Hanser Verlag München/Wien 1981.

Die pestiziden Zubereitungen enthalten in der Regel 0,1 bis 99%, insbesondere 0,1 bis 95%, des erfindungsgemässen Wirkstoffs oder Kombinationen dieser Wirkstoffe mit andern Insektiziden oder Akariziden, 1 bis 99,9% eines festen oder flüssigen Zusatzstoffes und 0 bis 25%, insbesondere 0,1 bis 20%, eines Tensides, wobei sich die %-Angaben auf das Gewicht beziehen. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1 :

a) Herstellung der Ausgangsverbindung 5-Aethoxy-6-acetyl-1,3-benzoxathiol :

Es werden 14,0 g wasserfreies Aluminiumchlorid und 18,2 g 5-Aethoxy1,3-benzoxathiol bei 0°C in 80 ml Methylenchlorid unter Argon vorgelegt. Dann tropft man bei 0°C unter Rühren 8,25 g Acetylchlorid zu dem Ansatz und rührt das Reaktionsgemisch bei 22°C während 16 Stunden. Die Aufarbeitung erfolgt durch Eingiessen in Eis-Wasser und Extraktion des wässrigen Gemisches mit Diäthyläther. Die organische Phase wird jeweils zweimal mit 100 ml Wasser und gesättigter Kochsalzlösung gewaschen und anschliessend über Natriumsulfat getrocknet. Nach Abdampfen des Lösungsmittels erhält man eine gelb-grüne feste Masse, die chromatographisch über Kieselgel mit Methylenchlorid/Essigester (10 : 1) als Elutionsmittel gereinigt wird. Das erhaltene feste Produkt wird anschliessend aus Essigester umkristallisiert.

Die sich auf diese Weise ergebende Titelverbindung hat einen Schmelzpunkt von 131-133°C.

b) Herstellung von [α-(4′-Methoxyphenyl)-α-(5-äthoxy-1,3-benzoxathiol-6-yl)]-äthylen :

Es werden 1,15 g Magnesiumspäne in 10 ml Tetrahydrofuran mit einer katalytischen Menge Jod in einer Argonatmosphäre vorgelegt. Nach Zugabe von wenig Bromanisol startet die Grignard-Reaktion und wird durch Zutropfen von 8,90 g Bromanisol am Rückfluss (67°C) gehalten. 5-Aethoxy-6-acetyl-1,3-benzoxathiols in 30 ml Tetrahydrofuran zugetropft und der Ansatz während 1.5 Stunden bei 67°C gehalten. Nach dem Abkühlen werden weitere 20 ml Tetrahydrofuran zugegeben. Der Ansatz wird während 1 Stunde bei 20°C nachgerührt und das Lösungsmittel abgezogen. Dann wird der Rückstand in Diäthyläther aufgenommen. Nach dem Waschen mit gesättigter Kochsalzlösung und Natriumthiosulfatlösung ergibt das Abdampfen des Lösungsmittels ein viskoses, grün-braunes Oel. Die Reinigung erfolgt mittels Säulenchromatographie an Kieselgel und mit Hexan/Methylenchlorid (2 : 3) als Elutionsmittel. Man erhält so die Titelverbindung der Formel

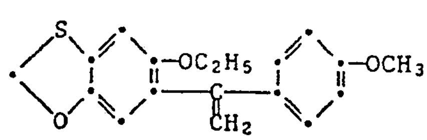

in kristalliner Form, Smp. 59-62°C (Verbindung Nr. 1).

Beispiel 2 : Herstellung von [α-(4′-Methoxyphenyl)-α-(5-äthoxy-1,3-benzdioxol-6-yl)]-äthylen :

Es werden 50 ml n-Butyllithium (1,6 molare Lösung in Hexan) in 25 ml Tetrahydrofuran bei −40°C (Aceton/Alkohol-Trockeneis-Bad) in einer Argonatmosphäre vorgelegt. Innerhalb von 10 Minuten werden 12,25 g 5-Aethoxy-6-brom-1,3-benzdioxol in 25 ml Tetrahydrofuran bei einer Temperatur unterhalb −30°C zugetropft. Der Ansatz wird dann während 30 Minuten bei −30 bis −40°C gerührt. Anschliessend werden zu dem Gemisch 7,5 g 4-Methoxyacetophenon in 50 ml Tetrahydrofuran innerhalb von 20 Minuten bei −30°C tropfenweise zugegeben und anschliessend wird bei −15°C während 1 Stunde nachgerührt. Durch Zugaben von 50 ml Wasser und 100 ml 2 N HCl-Lösung wird die Reaktion abgebrochen. Die organische Phase wird abgetrennt und die wässrige Phase dreimal mit je 100 ml Diäthyläther extrahiert. Die vereinigten Aetherphasen werden zweimal mit je 50 ml gesättigter Kochsalzlösung gewaschen und über Natriumsulfat getrocknet. Nach Abdampfen des Lösungsmittels am Rotationsverdampfer hinterbleibt ein braunes Oel, da über 200 g Kieselgel mit Methylenchorid/Hexan (2 : 3) als Elutionsmittel chromatographisch gereinigt wird. Man erhält auf diese Weise die Titelverbindung der Formel

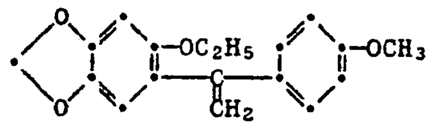

als weisse Kristalle, Smp. 69-72°C (Verbindung Nr. 2)

Analog der vorstehend aufgezeigten Arbeitsweise werden auch die folgenden Verbindungen der Formel Ia hergestellt :

| Verbindung Nr. | X | Y | $R_1$ | $R_2$ | $R_3$ | $R_4$ | $R_5$ | $R_6$ | physikal. Daten |
|---|---|---|---|---|---|---|---|---|---|
| 3 | -O- | -O- | $4-OCH_3$ | H | H | $-OCH_3$ | H | H | $n_D^{20}$ = 1,5915 |
| 4 | -O- | -O- | $4-OCH(CH_3)_2$ | H | H | $-OCH_3$ | H | H | $n_D^{20}$ = 1,5755 |
| 5 | -O- | -O- | $4-OCH(CH_3)_2$ | H | H | $-OC_2H_5$ | H | H | viskoses Oel (NMR) |
| 6 | -O- | -O- | $4-OC_2H_5$ | H | H | $-OC_2H_5$ | H | H | Smp. 68 - 72°C |
| 7 | -O- | -O- | $4-OCH_2C{\equiv}CH$ | H | H | $-OCH_3$ | H | H | viskoses Oel (NMR) |
| 8 | -O- | -O- | $6-OCH_3$ | H | H | $-OC_2H_5$ | H | H | Smp. 69 - 72°C |
| 9 | -O- | -O- | $5-OCH_3$ | H | H | $-OC_2H_5$ | H | H | viskoses Oel (NMR) |
| 10 | -O- | -O- | $4-OCH_3$ | H | H | $-OCH(CH_3)_2$ | H | H | Smp. 65 - 68°C |
| 11 | -O- | -O- | $4-OCH_3$ | H | H | $-OCH_2CH(CH_3)_2$ | H | H | Smp. 80 - 82°C |
| 12 | -O- | -O- | $4-OC_2H_5$ | H | H | $-OCH_3$ | H | H | Sdp. 190 - 200°C/ 0,1 Torr |
| 13 | -O- | -O- | $4-O(CH_2)_6CH_3$ | H | H | $-OC_2H_5$ | H | H | $n_D^{20}$ = 1,5545 |
| 14 | -O- | -O- | $4-O(CH_2)_2O(CH_2)_2OCH_3$ | H | H | $-OC_2H_5$ | H | H | viskoses Oel (NMR) |
| 15 | -O- | -O- | $4-OCH_3$ | H | H | $-O(CH_2)_2CH(CH_3)_2$ | H | H | viskoses Oel (NMR) |
| 16 | -O- | -O- | $4-OCH_3$ | H | H | $-O(CH_2)_2O(CH_2)_2OCH_3$ | H | H | Smp. 46 - 49°C |
| 17 | -O- | -O- | $4-OCH_3$ | H | H | $-O(CH_2)_6CH_3$ | H | H | Sdp. 150 - 200°C/ 0.1 Torr |
| 18 | -O- | -O- | $4-OCH_3$ | H | H | $-OCH_2C(CH_3)_3$ | H | H | viskoses Oel (NMR) |
| 19 | -O- | -O- | $4-OCH_3$ | H | H | $-OC_2H_5$ | $-CH_3$ | $-CH_3$ | Smp. 95 - 97°C |
| 20 | -O- | -O- | $4-OCH_3$ | H | H | $-OC_2H_5$ | $-CH_3$ | H | $n_D^{20}$ = 1,5835 |
| 21 | -O- | -O- | $4-OCF_2CHFCl$ | H | H | $-OC_2H_5$ | H | H | $n_D^{20}$ = 1,5560 |
| 22 | -O- | -O- | $4-OCH_3$ | H | H | $-OC_2H_5$ | $-CH_3$ | H | viskoses Oel (NMR) |
| 23 | -O- | -O- | $4-OCH_3$ | 5-F | H | $-OC_2H_5$ | H | H | Smp. 73 - 75°C |
| 24 | -O- | -O- | $4-O(CH_2)_2CH_3$ | H | H | $-OC_2H_5$ | H | H | $n_D^{20}$ = 1,5790 |

| Verbindung Nr. | X | Y | $R_1$ | $R_2$ | $R_3$ | $R_4$ | $R_5$ | $R_6$ | physikal. Daten |
|---|---|---|---|---|---|---|---|---|---|
| 25 | -O- | -O- | 4-$OCHF_2$ | H | H | -$OC_2H_5$ | H | H | Smp. 75 - 78°C |
| 26 | -O- | -O- | 4-$OCH_3$ | 5-F | H | -$OCH_3$ | H | H | Smp. 63 - 65°C |
| 27 | -O- | -O- | 4-$O(CH_2)_2CH_3$ | H | H | -$OCH_3$ | H | H | $n_D^{20}$ = 1,5910 |
| 28 | -O- | -O- | 4-$OCH_3$ | 5-F | H | -$O(CH_2)_6CH_3$ | H | H | Smp. 57 - 60°C |
| 29 | -O- | -O- | 4-$OCHF_2$ | H | H | -$OCH_3$ | -$CH_3$ | H | viskoses Oel (NMR) |
| 30 | -O- | -O- | 4-$OCH_3$ | H | H | -$O(CH_2)_6CH_3$ | -$CH_3$ | H | viskoses Oel (NMR) |
| 31 | -O- | -O- | 4-$OCH_3$ | 6-F | H | -$OC_2H_5$ | H | H | Smp. 44 - 47°C |
| 32 | -O- | -O- | 4-$OCH_3$ | 5-$OCH_3$ | H | -$OC_2H_5$ | H | H | Smp. 95 - 98°C |
| 33 | -O- | -O- | 4-$OCH_3$ | 3-$OCH_3$ | 5-$OCH_3$ | -$OC_2H_5$ | H | H | Smp. 70 - 71°C |
| 34 | -O- | -O- | 4,5-O-$CH_2$-$CH_2$-O- | | H | -$OC_2H_5$ | H | H | viskoses Oel (NMR) |
| 35 | -O- | -O- | 4,5-O-$CH_2$-O- | | H | -$OC_2H_5$ | H | H | $n_D^{20}$ = 1,6055 |
| 36 | -O- | -O- | 4,5-O-$CH_2$-O- | | H | H | H | H | Smp. 97 - 100°C |
| 37 | -O- | -O- | 3,4-O-$CH_2$-O- | | 6-$NO_2$ | -$OC_2H_5$ | H | H | Smp. 143 - 146°C |
| 38 | -O- | -O- | 6-$OC_2H_5$ | 3-Cl | 4-Cl | -$OC_2H_5$ | H | H | Smp. 73 - 76°C |
| 39 | -O- | -O- | 4-$OCH_3$ | 3-F | 5-F | -$OC_2H_5$ | H | H | Smp. 51 - 53°C |
| 40 | -O- | -O- | 4-$OCH_3$ | 3-F | 6-F | -$OC_2H_5$ | H | H | Smp. 91 - 93°C |
| 41 | -O- | -O- | 6-$OCH_3$ | 3-F | H | -$OC_2H_5$ | H | H | Smp. 75 - 79°C |
| 42 | -O- | -O- | 6-$OCH_3$ | 3-F | 5-F | -$OC_2H_5$ | H | H | $n_D^{20}$ = 1,5400 |
| 43 | -O- | -O- | 4-$OCH_3$ | H | H | H | H | H | Smp. 66 - 67°C |
| 44 | -S- | -O- | 4-$OCH_3$ | 3-F | H | -$OC_2H_5$ | H | H | $n_D^{20}$ = 1,6180 |
| 45 | -O- | -O- | 4-$OCH_3$ | 2-$OCH_3$ | H | -$OC_2H_5$ | H | H | $n_D^{20}$ = 1,5905 |
| 46 | -O- | -O- | H | 4-F | H | -$OC_2H_5$ | H | H | Smp. 66-67°C |
| 47 | -O- | -O- | H | 3-F | 5-F | -$OC_2H_5$ | H | H | $n_D^{20}$ = 1,5585 |
| 48 | -O- | -O- | H | 2-F | 4-F | -$O_2H_5$ | H | H | Smp. 73-74°C |
| 49 | -O- | -O- | H | 2-F | 6-F | -$OC_2H_5$ | H | H | Smp. 87-89°C |

| Verbindung Nr. | X | Y | $R_1$ | $R_2$ | $R_3$ | $R_4$ | $R_5$ | $R_6$ | physikal. Daten |
|---|---|---|---|---|---|---|---|---|---|
| 50 | -O- | -O- | H | 2-F | H | $-OC_2H_5$ | H | H | $n_D^{20}$ = 1,5725 |
| 51 | -O- | -O- | H | $3-OCH_3$ | $5-OCH_3$ | $-OC_2H_5$ | H | H | Smp. 67-70°C |
| 52 | -O- | -O- | H | 3-F | H | $-OC_2H_5$ | H | H | $n_D^{20}$ = 1,5780 |
| 53 | -O- | -O- | H | 2-F | 5-F | $-OC_2H_5$ | H | H | $n_D^{20}$ = 1,5690 |
| 54 | -O- | -O- | $4-OCH_3$ | 3-Cl | H | $-OC_2H_5$ | H | H | Smp. 61-64°C |
| 55 | -O- | -O- | $4-OCH_3$ | $3-CH_3$ | H | $-OC_2H_5$ | H | H | $n_D^{20}$ = 1,5925 |
| 56 | -O- | -O- | $2-OC_2H_5$ | $6-OC_2H_5$ | H | $-OC_2H_5$ | H | H | Smp. 116-119°C |
| 57 | -O- | -O- | $2-OCH_3$ | $5-OCH_3$ | H | $-OC_2H_5$ | H | H | Smp. 72-74°C |
| 58 | -O- | -O- | $4-OCF_3$ | H | H | $-OC_2H_5$ | H | H | Smp. 63-65°C |
| 59 | -O- | -O- | $4-OCH_3$ | 2-F | 6-F | $-OC_2H_5$ | H | H | Sdp. 190°C/0,2 Torr |
| 60 | -O- | -O- | $4-OCH_3$ | 2-F | 3-F | $-OC_2H_5$ | H | H | Smp. 108-109°C |
| 61 | -O- | -O- | $4-OCH_3$ | H | H | $-C_3H_7(n)$ | H | H | Sdp. 180-185°C/ 0,02 Torr |
| 62 | -O- | -O- | $4-OCH_3$ | H | H | $-CH_2-OCH_3$ | H | H | Smp. 39-41°C |
| 63 | -O- | -O- | $4-OCH_3$ | H | H | $-CH_3$ | H | H | $n_D^{20}$ = 1,5930 |
| 64 | -O- | -O- | $4-OCH_3$ | 3-F | 5-F | $-CH_3$ | H | H | viskoses Oel (NMR) |
| 65 | -S- | -O- | $4-OCF_3$ | H | H | $-OC_2H_5$ | H | H | Smp. 57 - 60°C |
| 66 | -O- | -O- | H | 4-F | H | $-OC_2H_5$ | H | H | Smp. 109,5-111,5°C |
| 67 | -O- | -O- | $4-OCH_3$ | H | H | $-OC_2H_5$ | Cl | H | Smp. 134-136°C |
| 68 | -O- | -O- | $4-OCH_3$ | 3-F | H | $-C_3H_7(n)$ | H | H | $n_D^{20}$ = 1,5760 |
| 69 | -S- | -O- | $4-OCH_3$ | H | H | $-OCH_3$ | H | H | Smp. 91-93,5°C |
| 70 | -S- | -O- | $4-OCH_3$ | 3-F | H | $-OCH_3$ | H | H | Smp. 115-118°C |

In entsprechender Weise — wie vorangehend angegeben — sind auch die folgenden Verbindungen der Formel I herstellbar:

| X | Y | $R_1$ | $R_2$ | $R_3$ | $R_4$ | $R_5$ | $R_6$ |
|---|---|---|---|---|---|---|---|
| -O- | -O- | 4-$OCH_3$ | H | H | $-OC_2H_5$ | F | F |
| -O- | -O- | 4-$OCH_3$ | H | H | $-OC_2H_5$ | F | H |
| -O- | -O- | 4-$OCH_3$ | H | H | $-OC_3H_7$(n) | H | H |
| -O- | -O- | 4-$OCH_3$ | H | H | $-OCH_2C{\equiv}CH$ | H | H |
| -O- | -O- | 4-$OCH_3$ | H | H | $-OCH_2CH{=}CH_2$ | H | H |
| -O- | -O- | 4-$OCH_3$ | H | H | $-OC_2H_5$ | Cl | Cl |
| -S- | -O- | 4-$OCH_3$ | H | H | $-OC_2H_5$ | F | F |

Beispiel 3:

Formulierungen für flüssige Wirkstoffe der Formel I gemäss Beispiel 1 bzw. 2 oder Kombinationen dieser Wirkstoffe mit andern Insektiziden oder Akariziden (% = Gewichtsprozent)

| 1. Emulsions-Konzentrate | a) | b) | c) |
|---|---|---|---|
| Wirkstoff resp. Wirkstoffkombination | 25 % | 40 % | 50 % |
| Ca Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexan | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff resp. Wirkstoffkombination | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 3. Granulate | a) | b) |
|---|---|---|
| Wirkstoff resp. Wirkstoffkombination | 5 % | 10 % |
| Kaolin | 94 % | - |
| Hochdisperse Kieselsäure | 1 % | - |
| Attapulgit | - | 90 % |

Der Wirkstoff bzw. die Wirkstoffkombination wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff resp. Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff bzw. der Wirkstoffkombination erhält man gebrauchsfertige Stäubemittel.

Formulierungen für feste Wirkstoffe der Formel I gemäss Beispiel 1 bzw. 2 resp. Kombinationen dieser Wirkstoffe mit andern Insektiziden oder Akariziden (% = Gewichtsprozent)

| 1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2. Emulsions-Konzentrat | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentra-

tion hergestellt werden.

| 3. Stäubemittel | a) | b) |
| --- | --- | --- |
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

| 4. Extruder-Granulat | |
| --- | --- |
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

| 5. Umhüllungs-Granulat | |
| --- | --- |
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

| 6. Suspensions-Konzentrat | |
| --- | --- |
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder der Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

7. <u>Aufguss-Lösung ("pour-on"-Zubereitung)</u>

| | |
|---|---|
| Wirksubstanz | 30,00 g |
| Natrium-dioctylsulfosuccinat | 3,00 g |
| Benzylalkohol | 35,46 g |
| Ethylenglykol-monomethylether | 35,46 g |
| | 103,92 g = 100 ml |

Die Wirksubstanz wird in dem grössten Teil des Gemisches der beiden Lösungsmittel unter kräftigem Rühren, gelöst. Anschliessend wird das Natrium-dioctylsulfosuccinat, eventuell unter Erwärmen, gelöst und schliesslich mit dem restlichen Lösungsmittelgemisch aufgefüllt.

<u>Beispiel 4 : Wirkung gegen Musca domestica :</u>

Je 50 g frisch zubereitetes Nährsubstrat für Maden werden in Becher eingewogen. Von einer 1 Gew.-% igen acetonischen Lösung des betreffenden Wirkstoffes wird eine bestimmte Menge auf das in den Bechern befindliche Nährsubstrat pipettiert, so dass sich eine Wirkstoffkonzentration von 800 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert. Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss den Beispielen 1 und 2 zeigen gute Wirkung im obigen Test.

<u>Beispiel 5 : Wirkung gegen Lucilia sericata :</u>

Zu 9 ml eines Zuchtmediums wird bei 50°C 1 ml einer 100 ppm Aktivsubstanz enthaltenden wässrigen Zubereitung hinzugefügt. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Die Verbindung Nr. 2 gemäss Beispiel 2 zeigt in diesem Test 80-100% Wirkung gegen Lucilia sericata.

<u>Beispiel 6 : Wirkung gegen Lucilia cuprina</u>

Frisch abgelegte Eier der Schmeissfliege L.cuprina werden in kleinen Portionen (30-50 Eier) in Reagenzgläser gegeben, in denen zuvor 4 ml Nährmedium mit 1 ml Wirkstofflösung vermischt worden sind. Nach der Beimpfung des Kulturmediums werden die Testgläser mit einem Wattestopfen verschlossen und im Brutschrank bei 30°C über 4 Tage bebrütet.

In dem zum Vergleich unbehandelten Medium entwickeln sich bis zu diesem Zeitpunkt ca. 1 cm lange Larven (Stadium 3). Ist eine Substanz aktiv, so sind die Larven zu diesem Zeitpunkt entweder tot oder moribund. Der Versuch wird mit einer Wirkstoff-Konzentration von 100 ppm durchgeführt. Auch Repellenz wird berücksichtigt, wenn die Larven aus dem Medium auswandern und verhungern.

Die Verbindung Nr. 1 gemäss Beispiel 1 zeigt in diesem Test 80-100% Wirkung gegen Lucilia cuprina.

<u>Beispiel 7 : Wirkung gegen Aëdes aegypti :</u>

Auf die Oberfläche von 150 ml Wasser, das sich in einem Behälter befindet, wird so viel einer 0,1 Gew.-%igen acetonischen Lösung des Wirkstoffes pipettiert, dass eine Konzentration von 10 ppm erhalten wird. Nach Verdunsten des Acetons wird der Behälter mit 30 bis 40 2-tägigen Aëdes-Larven beschickt. Nach 8 Tagen wird die Mortalität geprüft.

Die Verbindung Nr. 2 gemäss Beispiel 2 zeigt in diesem Test 100% Wirkung (Mortalität) gegen Aëdes aegypti.

Beispiel 8 : Wirkung gegen Anthonomus grandis (Adulte)

Zwei eingetopfte Baumwollpflanzen im 6-Blattstadium werden jeweils mit einer wässrigen benetzungsfähigen Emulsions-Zubereitung enthaltend 400 ppm des zu prüfenden Wirkstoffes besprüht. Nach dem Antrocknen des Spritzbelages (nach etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25°C und etwa 60% relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen der Formel I gemäss Beispiel 2 zeigen gute Wirkung in obigem Test.

Beispiel 9 : Insektizide Wirkung : Nilaparvata lugens

Reispflanzen werden mit einer Versuchslösung, enthaltend 400 ppm der zu prüfenden Verbindung, besprüht. Nach dem Antrocknen des Belages werden die Pflanzen mit Nymphen von Nilaparvata lugens (N2 oder N3-Stadium) besetzt. Man verwendet pro Versuchsverbindung und pro Test-Spezies zwei Pflanzen. Die Auswertung der erzielten prozen tualen Abtötungsrate erfolgt nach 6 Tagen. Der Versuch wird bei 26°C und 60% relativer Luftfeuchtigkeit durchgeführt.

Verbindung Nr. 2 zeigt im obigen Test 80-100%-ige Wirkung gegenüber Nilaparvata lugens-Nymphen.

Beispiel 10 : Wirkung gegen Bodeninsekten (Diabrotica balteata)

Es werden 5 Maiskeimlinge von 1-3 cm sowie eine Filterpapier-Rondelle in eine wässrige Wirkstofflösung enthaltend etwa 4 Vol.-% Aceton eingetaucht. Die eingetauchte Filterpapier-Rondelle wird auf den Boden eines Kunststoff-Bechers (Inhalt 200 ml) gelegt und darauf wird eine trockene Filterpapier-Rondelle sowie die Maiskeimlinge und 10 Larven von Diabrotica balteata im 2. oder 3. Larvalstadium gegeben. Der Ansatz wird bei ca. 24°C und 40-60% relativer Luftfeuchtigkeit und Tageslicht gehalten. Die Bonitur erfolgt nach 6 Tagen gegenüber unbehandelten Kontrollansätzen.

Die erfindungsgemässen Verbindungen Nr. 1 und 2 zeigen in diesem Test bei 400 ppm eine Wirkung von 80-100% (Mortalität).

Beispiel 11 : Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 50 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23°C, bei 55% relativer Luftfeuchtigkeit und mit einer Belichtungsperiode von 16 Stunden durchgeführt.

Die erfindungsgemässe Verbindung Nr. 2 gemäss Beispiel 2 zeigt in diesem Test 80-100%-ige Wirkung.

Beispiel 12 : Insektizide Frassgift-Wirkung gegen Plutella xylostella :

Eingetopfte Chinakohlpflanzen (Topfgrösse 10 cm Durchmesser) im 4-Blatt-Stadium werden mit wässrigen Wirkstoffemulsionen besprüht, die den Wirkstoff in Konzentrationen von 50 bis 400 ppm enthalten und auf den Pflanzen antrocknen.

Nach zwei Tagen werden die behandelten Chinakolhpflanzen mit je 10 Plutella xylostella-Larven im zweiten bis dritten larvalen Stadium besiedelt. Der Versuch wird bei 24°C und 60% relativer Luftfeuchtigkeit im Dämmerlicht durchgeführt. Die Bonitur erfolgt nach 2 und 5 Tagen ; es wird die %-Mortalität der Larven bestimmt.

Eine Wirkung von 100% (Mortalität) bei 50 ppm zeigt die Verbindung Nr. 1 gemäss Beispiel 1 in diesem Test.

Beispiel 13 : Insektizide Kontaktwirkung : Aphis craccivora

In Töpfen angezogene Bohnen-Pflanzen (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe besprüht. Man verwendet für die zu prüfende Verbindung je zwei Pflanzen : die Auswertung der erzielten Abtötungsrate erfolgt nach weiteren 24 Stunden.

Die Verbindungen Nr. 1 und 2 zeigen 80-100%ige Wirkung (Mortalität) in diesem Test.

Beispiel 14 : Ovizide Wirkung auf Heliothis virescens und Spodoptera littoralis

Entsprechende Mengenanteile einer emulgierbaren Formulierung, enthaltend 25 Gew.-% des zu prüfenden Wirkstoffes, werden mit jeweils soviel Wasser vermischt, dass sich wässrige Emulsionen von ansteigender Wirkstoffkonzentration von 400 bis 800 ppm ergeben.

In diese wirkstoffhaltigen Emulsionen werden eintägige Eigelege von Heliothis auf Cellophan bzw. Eigelege von Spodoptera auf Fliesspapier während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen (Heliothis) bzw. 3 bis 4 Tagen (Spodoptera) wird die Schlupfrate im Vergleich zu unbehandelten Kontrollen festgestellt. Zur Auswertung wird die zur 100%-igen Abtötung der Eier erforderliche minimale Wirkstoffkonzentration bestimmt.

Verbindunge der Formel I gemäss Beispiel 1 bzw. 2 zeigen in diesem Test gute ovizide Wirkung gegen die geprüften Schädlinge. So zeigt Verbindung Nr. 2 schon bei 400 ppm 80-100%ige ovizide Wirkung gegen Heliothis und Spodoptera.

Beispiel 15 : Wirkung gegen pflanzenschädigende Akariden Tetranychus urticae (OP-sensible) und Tetranychus cinnabarinus (OP-tolerant).

Die Primärblätter von Phaseolus vulgaris-Pflanzen werden 16 Stunden vor dem Versuch auf akarizide Wirkung mit einem infestierten Blattstück aus einer Massenzucht von Tetranychus urticae (OP-sens.) bzw. Tetranychus cinnabarinus (OP-tol.) belegt (Die Toleranz bezieht sich auf die Verträglichkeit gegenüber Diazinon). Die so behandelten infestierten Pflanzen werden dann mit einer Versuchslösung enthaltend 400 ppm der zu prüfenden Verbindung bis zur Tropfnässe besprüht. Nach 24 Stunden und wiederum nach 7 Tagen werden Imagines und Larven (alle beweglichen Stadien) unter dem Binokular auf lebende und tote Individuen ausgewertet.

Man verwendet pro Konzentration und pro Testspezies ein Pflanze. Während des Versuchsverlaufs stehen die Pflanzen in Gewächshauskabinen bei 25°C.

Verbindungen der Formel I gemäss den Beispielen 1 und 2 zeigen in diesem Versuch gute Wirkung gegen Tetranychus urticae und Tetranychus cinnabarinus. So ergibt die Verbindung Nr. 2 eine 100%ige Abtötung gegen Tetranychus urticae.

Beispiel 16 : Ovizide Wirkung auf Tetranychus urticae (OP-resistent)

Eingetopfte Phaseolus vulgaris-Pflanzen im Primärblatt-Stadium werden jeweils zweimal mit 30 Weibchen von Tetranychus urticae besiedelt. Nach 24-stündiger Eiablage werden die Weibchen mit einer Saugpumpe (Wasserstrahlpumpe) von den Pflanzen entfernt, so dass auf diesen nur noch die abgelegten Milbeneier verbleiben.

Die mit den Milbeneiern infestierten Pflanzen werden dann mit einer wässrigen Emulsion enthaltend 200 bis 400 ppm der zu prüfenden Verbindung bis zur Tropfnässe besprüht und 5 Tage bei 25°C und etwa 50% relative Luftfeuchtigkeit gehalten. Nach dieser Zeit wird die prozentuale Mortalität der Eier und geschlüpften Larven durch Auszählen bestimmt.

Verbindungen der Formel I gemäss den vorstehenden Beispielen 1 und 2 zeigen gute Wirkung in obigem Test. So ergibt die Verbindung Nr. 2 80-100%ige Wirkung (Mortalität) schon bei 200 ppm.

Beispiel 17 : Chemosterilisierende Wirkung auf Anthonomus grandis

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 400 bis 1000 ppm des zu prüfenden Wirkstoffes, eingetaucht.

Wenn die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des Chemosterilans-Effektes der zu prüfenden Wirkstoffe wird die Eiablage der Käfer während eines Zeitraumes von etwa vier Wochen überprüft. Die Bonitierung erfolgt anhand der Verminderung der Anzahl abgelegter Eier und geschlüpfter Larven im Vergleich zu unbehandelten Kontrollen.

Verbindungen der Formel I gemäss Beispiel 1 bzw. 2 zeigen gute Wirkung im obigen Test. So ergibt die Verbindung Nr. 2 eine 80-100%ige Wirkung schon bei 400 ppm.

Beispiel 18 : Insektizide Frassgift-Wirkung

Ca. 25 cm hohe eingetopfte Baumwollpflanzen werden mit einer wässrigen Wirkstoffemulsion besprüht, die den jeweiligen Wirkstoff in einer Konzentration von 400 ppm enthält.

Nach dem Antrocknen des Sprühbelages werden pro Konzentration je 3 Baumwollpflanzen in einem Metallkessel gestellt und mit 50 Spodoptera littoralis-Larven im ersten larvalen Stadium besiedelt. Der Kessel wird dann mit einer Glasplatte abgedeckt. Der Versuch wird bei 28°C und etwa 60% relativer Luftfeuchtigkeit durchgeführt. Nach 96 Stunden wird die %-Mortalität der Test-Insekten gegenüber unbehandelten Kontrollen bestimmt.

Verbindung Nr. 1 gemäss Beispiel 1 zeigt eine Wirkung von 80-100% (Mortalität) in diesem Test.

Beispiel 19 : Wirkung gegen Zecken

Als Testtiere werden mit Blut vollgesogene adulte Weibchen der Rinderzecke Boophilus microplus verwendet. Es werden je 10 Zecken eines OP-resistenten Stammes (z.B. vom Biarra-Stamm) und eines normal empfindlichen Stammes (z.B. vom Yeerongpilly-Stamm) behandelt. Die Zecken werden auf mit Doppelklebeband bezogenen Platten dorsal fixiert und dann mit einem mit wässrigen Emulsionen bzw. Lösungen enthaltend 400 ppm der zu untersuchenden Verbindung getränkten Wattebausch während 1 Stunde bedeckt. Nach Entfernung des Wattebausches werden die Zecken über Nach bei 24°C getrocknet und anschliessend in einem klimatisierten Raum bei konstanten Bedingungen (28°C, 80% rel. Luftfeuchtigkeit) gehalten, und zwar für 4 Wochen bis zum Ende der Eiablage und Beginn des Larvenschlupfes. Zur Auswertung wird die Mortalität, die prozentuale Hemmung der Ablage von fertilen Eiern (Blockierung der Embryogenese bzw. des Schlupfes) gegenüber unbehandelten Kontrollen ermittelt.

Die Verbindungen Nr, 1, 2, 3, 6, 10, 12, 23, 26, 31, 40, 44, 55 und 56 gemäss den Beispielen 1 und 2 zeigen 80-100% Wirkung in obigem Test.

Beispiel 20 : Wirkung gegen Zecken : Abtötungswirkung in verschiedenen Entwicklungsstadien

Als Testobjekte werden Larven (jeweils ca. 50), Nymphen (jeweils ca. 25) oder Imagines (jeweils ca. 20) der Zeckenarten Rhipicephalus bursa, Amblyomma hebraeum und Boophilus microplus verwendet. Die Testtiere werden für kurze Zeit in wässrige Emulsionen der zu untersuchenden Substanzen mit einer Konzentration von 800 ppm getaucht. Die in Teströhrchen befindlichen Emulsionen werden dann mit Watte aufgenommen und die benetzten Testtiere in den so kontaminierten Röhrchen belassen. Die Auswertung (% — Mortalität) erfolgt für Larven nach 3 Tagen und für Nymphen und Imagines nach 14 Tagen.

Verbindungen der Formel I gemäss den Beispielen 1 und 2 sind in diesem Test gut wirksam.

## Patentansprüche

1. Verbindung der Formel I

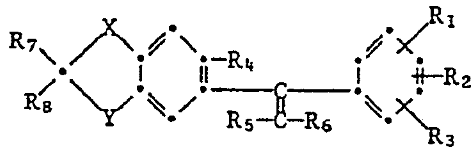

(I),

worin

| | |
|---|---|
| $R_1$ und $R_4$ | unabhängig voneinander Wasserstoff, Hydroxy, $C_1$-$C_5$-Alkyl, $C_1$-$C_8$-Alkoxy, mit 1 bis 7 Halogenatomen substituiertes $C_1$-$C_3$-Alkoxy, Alkoxyalkoxy mit insgesamt 2 bis 6 C-Atomen, $C_3$-$C_5$-Alkenyloxy oder $C_3$-$C_5$-Alkinyloxy ; |
| $R_2$ und $R_3$ | unabhängig voneinander Wasserstoff, Halogen, $C_1$-$C_3$-Alkyl, $C_1$-$C_5$-Alkoxy oder Nitro ; oder |
| $R_1$ und $R_2$ | zusammen einen Rest —O-$CH_2$-O— oder —O-$CH_2$-$CH_2$-O— ; |
| $R_5$ und $R_6$ | unabhängig voneinander Wasserstoff, Halogen oder Methyl ; |
| $R_7$ und $R_8$ | unabhängig voneinander Wasserstoff, Methyl oder Aethyl ; |
| | und X und Y unabhängig voneinander —O— oder —S— bedeuten, mit der Maßgabe, daß mindestens einer der Substituenten $R_1$ bis $R_8$ eine andere Bedeutung als Wasserstoff hat, wenn X und Y beide für —O— stehen. |

2. Verbindung der Formel I gemäss Anspruch 1, dadurch gekennzeichnet, dass

| | |
|---|---|
| $R_1$ und $R_4$ | unabhängig voneinander Wasserstoff, $C_1$-$C_8$-Alkoxy, mit 1 bis 7 Halogenatomen substituiertes $C_1$-$C_3$-Alkoxy, Alkoxyalkoxy mit insgesamt 2 bis 6 C-Atomen, $C_3$-$C_5$-Alkenyloxy oder $C_3$-$C_5$-Alkinyloxy ; |
| $R_2$ und $R_3$ | unabhängig voneinander Wasserstoff, Halogen, $C_1$-$C_3$-Alkyl, $C_1$-$C_5$-Alkoxy oder Nitro ; oder |
| $R_1$ und $R_2$ | zusammen einen Rest —O-$CH_2$-O— oder —O-$CH_2$-$CH_2$-O— ; |
| $R_5$ und $R_6$ | unabhängig voneinander Wasserstoff, Halogen oder Methyl ; |
| $R_7$ und $R_8$ | unabhängig voneinander Wasserstoff, Methyl oder Aethyl ; |

X —O— oder —S— ; und Y —O— bedeuten, mit der Maßgabe, daß mindestens einer der Substituenten $R_1$ bis $R_8$ eine andere Bedeutung als Wasserstoff hat, wenn X und Y beide für —O— stehen.

3. Verbindung gemäss Anspruch 2 der Formel Ia

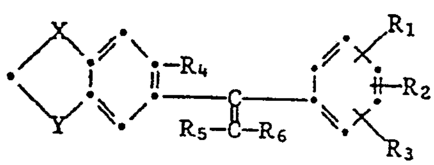

(Ia),

worin $R_1$ bis $R_6$, X und Y die in Anspruch 2 angegebenen Bedeutungen haben.

4. Verbindung gemäss Anspruch 2 der Formel Ib

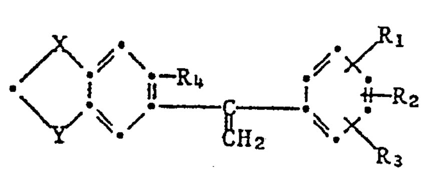

(Ib),

worin $R_1$ bis $R_4$, X und Y die in Anspruch 2 angegebenen Bedeutungen haben.

5. Verbindung gemäss einem der Ansprüche 1 bis 4, dadurch gekennzeichnet, dass

| | |
|---|---|
| $R_1$ und $R_4$ | unabhängig voneinander Wasserstoff, $C_1$-$C_5$-Alkoxy, mit 1 bis 5 Fluor- oder Chloratomen substituiertes $C_1$-$C_2$-Alkoxy, Alkoxyalkoxy mit insgesamt 2 bis 5 C-Atomen oder Propinyloxy ; |
| $R_2$ und $R_3$ | unabhängig voneinander Wasserstoff, Fluor, Chlor, Methoxy oder Aethoxy ; oder |
| $R_1$ und $R_2$ | zusammen den Rest —O-$CH_2$-O— oder —O-$CH_2$-$CH_2$-O— ; und |
| $R_5$ und $R_6$ | unabhängig voneinander Wasserstoff, Halogen oder Methyl |

bedeuten, mit der Maßgabe, daß mindestens einer der Substituenten $R_1$ bis $R_8$ eine andere Bedeutung als Wasserstoff hat, wenn X und Y beide für —O— stehen.

6. Verbindung der Formel Ib gemäss Anspruch 4, dadurch gekennzeichnet, dass
$R_1$ Methoxy, Aethoxy oder Trifluormethoxy ;
$R_2$ und $R_3$ unabhängig voneinander Wasserstoff oder Fluor ; und
$R_4$ $C_1$-$C_8$-Alkoxy
bedeuten.

7. Verbindung gemäss einem der Ansprüche 1 bis 6 der Formel Ic

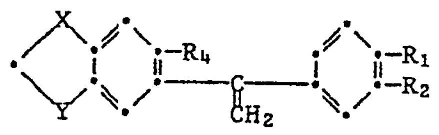

(Ic),

wobei $R_1$, $R_2$ und $R_4$ sowie X und Y die in den Ansprüchen 1 bis 6 angegebenen Bedeutungen unter Einbezug der gegebenen Maßgabe besitzen.

8. Verbindung gemäss einem der Ansprüche 1 bis 7, dadurch gekennzeichnet, dass X und Y gleichzeitig —O— unter Einbezug der gegebenen Maßgabe bedeuten.

9. Verbindung gemäss einem der Ansprüche 1 bis 7, dadurch gekennzeichnet, dass X —S— und Y —O— bedeuten.

10. Verbindung gemäss einem der Ansprüche 1 bis 9, dadurch gekennzeichnet, dass $R_2$ Fluor bedeutet.

11. Verbindung gemäss Anspruch 8 der Formel

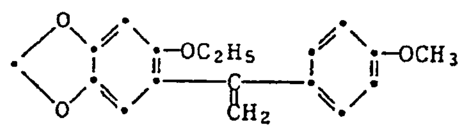

.

12. Verbindung gemäss Anspruch 8 der Formel

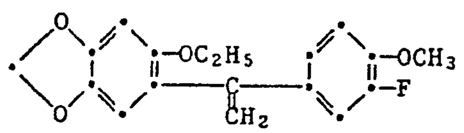

.

13. Verbindung gemäss Anspruch 8 der Formel

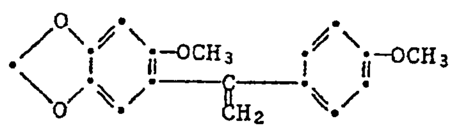

.

14. Verbindung gemäss Anspruch 9 der Formel

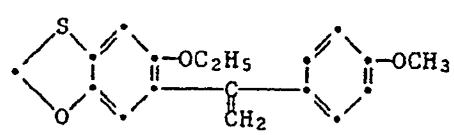

15. Verbindung gemäss Anspruch 8 der Formel

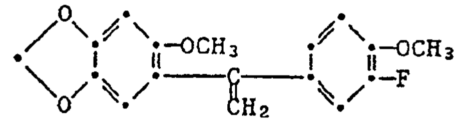

16. Verbindung gemäss Anspruch 9 der Formel

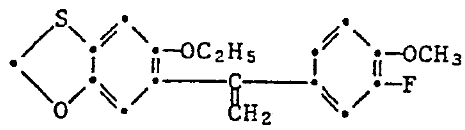

17. Verbindung gemäss Anspruch 9 der Formel

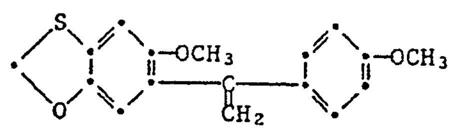

18. Verbindung gemäss Anspruch 9 der Formel

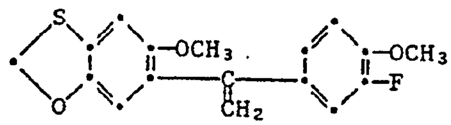

19. Verfahren zur Herstellung einer Verbindung der Formel 1 gemäss einem der Ansprüche 1 bis 18, dadurch gekennzeichnet, dass man eine Verbindung der Formel II

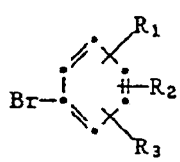

(II)

in einer Grignard-Reaktion in Gegenwart von Magnesium oder unter Einwirkung eines Lithiumalkyls mit einer Verbindung der Formel III

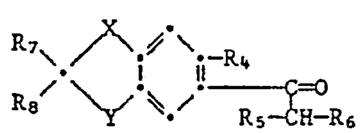

(III),

umsetzt, wobei in den Formeln II und III $R_1$ bis $R_8$, X und Y die unter Anspruch 1 bis 10 angegebenen Bedeutungen haben.

20. Verfahren zur Herstellung einer Verbindung der Formel I, worin X und Y gleichzeitig —O— bedeuten, gemäss einem der Ansprüche 1 bis 8, 10 bis 13 und 15, dadurch gekennzeichnet, dass man eine Verbindung der Formel IV.

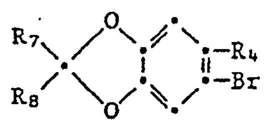

(IV)

in einer Grignard-Reaktion in Gegenwart von Magnesium oder unter Einwirkung eines Lithiumalkyls mit einer Verbindung der Formel V

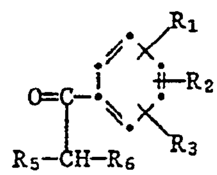

(V)

umsetzt, wobei in den Formeln IV und V $R_1$ bis $R_8$ die unter Anspruch 1 bis 7 und 10 angegebenen Bedeutungen haben.

21. Verbindung der Formel III gemäss Anspruch 19 mit der Maßgabe, daß in denjenigen Verbindungen der Formel III, in denen

$R_4$ Wasserstoff, Hydroxy, $C_1$-$C_5$ -Alkyl, $C_1$-$C_8$-Alkoxy, mit 1 bis 7 Halogenatomen substituiertes $C_1$-$C_3$ -Alkoxy, Alkoxyalkoxy mit insgesamt 2 bis 6 C-Atomen, $C_3$-$C_5$ -Alkenyloxy oder $C_3$-$C_5$-Alkinyloxy,

$R_5$ und $R_6$ unabhängig voneinander Wasserstoff, Halogen oder Methyl,

$R_7$ und $R_8$ unabhängig voneinander Wasserstoff, Methyl oder Aethyl und

X und Y unabhängig voneinander —O— oder —S—

bedeuten, mit der Massgabe, dass in denjenigen Verbindungen der Formel III, in denen

i) $R_4$, $R_7$ und $R_8$ Wasserstoff und X und Y beide —O— bedeuten und einer der beiden Substituenten $R_5$ und $R_6$ für Wasserstoff steht, der andere dieser beiden Substituenten für Halogen steht;

ii) $R_4$ Hydroxy oder Methoxy, $R_7$ und $R_8$ Wasserstoff und X und Y beide —O— bedeuten, mindestens einer der beiden Substituenten $R_5$ und $R_6$ für Halogen oder Methyl steht;

iii) $R_4$ Wasserstoff und von den beiden Substituenten $R_5$ und $R_6$ der eine Wasserstoff und der andere Chlor bedeutet und X für —O— und Y für —S— steht, von den Substituenten $R_7$ und $R_8$ entweder mindestens einer für Wasserstoff oder einer für Methyl und der andere für Aethyl steht;

iv) $R_4$, $R_5$ und $R_6$ Wasserstoff bedeuten und X für —O— und Y für —S— steht, von den beiden Substituenten $R_7$ und $R_8$ der eine Wasserstoff und der andere Methyl oder Aethyl bedeutet.

22. Verfahren zur Herstellung einer Verbindung der Formel III gemäss Anspruch 21, dadurch gekennzeichnet, dass man eine Verbindung der Formel VIII

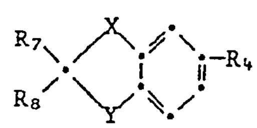

(VIII)

in Gegenwart eines Friedel-Crafts-Katalysators mit einem Acylhalogenid der Formel IX

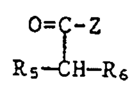

(IX)

umsetzt, wobei $R_4$ bis $R_8$, X und Y die unter Anspruch 1 bis 10 angegebenen Bedeutungen haben und Z ein Halogenatom, vorzugsweise Chlor, bedeutet.

23. Schädlingsbekämpfungsmittel, welches als aktive Komponente eine Verbindung der Formel I gemäss einem der Ansprüche 1 bis 18 zusammen mit geeigneten Trägern und/oder anderen Zuschlagstoffen enthält.

24. Verwendung einer Verbindung der Formel I gemäss einem der Ansprüche 1 bis 18 zur Bekämpfung von Insekten und Vertretern der Ordnung Akarina an Pflanzen.

25. Verwendung gemäss Anspruch 24 zur Bekämpfung larvaler Stadien pflanzenschädigender fressender Insekten.

26. Verwendung gemäss Anspruch 24 zur Bekämpfung pflanzenschädigender saugender Insekten.

27. Verbindung der Formel I gemäss den Ansprüchen 1 bis 18 zur Anwendung bei den Bekämpfung von Ektoparasiten an Haus- und Nutztieren.

28. Verbindung gemäss Anspruch 27 zur Anwendung bei der Bekämpfung von Zecken.

29. Verfahren zur Bekämpfung von Insekten und Vertretern der Ordnung Akarina, dadurch gekennzeichnet, dass man diese Schädlinge bzw. deren verschiedene Entwicklungsstadien oder deren Aufenthaltsort mit Ausnahme menschlicher oder tierischer Körper mit einer pestizid wirksamen Menge einer Verbindung der Formel I gemäss einem der Ansprüche 1 bis 18 oder mit einem Mittel enthaltend neben Zusatz- und Trägerstoffen eine pestizid wirksame Menge dieser Verbindung, in Kontakt bringt oder behandelt.

## Claims

1. A compound of formula I

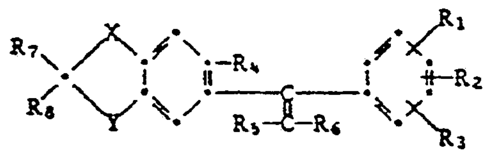

(I)

in which

$R_1$ and $R_4$ independently of one another each represents hydrogen, hydroxy, $C_1$-$C_5$alkyl, $C_1$-$C_8$alkoxy, $C_1$-$C_3$alkoxy substituted by from 1 to 7 halogen atoms, alkoxyalkoxy having a total of from 2 to 6 carbon atoms, $C_3$-$C_5$alkenyloxy or $C_3$-$C_5$alkynyloxy ;

$R_2$ and $R_3$ independently of one another each represents hydrogen, halogen, $C_1$-$C_3$alkyl, $C_1$-$C_5$alkoxy or nitro ; or

$R_1$ and $R_2$ together represent a radical $-O-CH_2-O-$ or $-O-CH_2-CH_2-O-$ ;

$R_5$ and $R_6$ independently of one another each represents hydrogen, halogen or methyl ;

$R_7$ and $R_8$ independently of one another each represents hydrogen, methyl or ethyl ; and

X and Y independently of one another each represents $-O-$ or $-S-$, with the proviso that at least one of the substituents $R_1$ to $R_8$ is other than hydrogen when X and Y both represent $-O-$.

2. A compound of formula I according to claim 1, characterised in that

$R_1$ and $R_4$ independently of one another each represents hydrogen, $C_1$-$C_8$alkoxy, $C_1$-$C_3$alkoxy substituted by from 1 to 7 halogen atoms, alkoxyalkoxy having a total of from 2 to 6 carbon atoms, $C_3$-$C_5$alkenyloxy or $C_3$-$C_5$alkynyloxy ;

$R_2$ and $R_3$ independently of one another each represents hydrogen, halogen, $C_1$-$C_3$alkyl, $C_1$-$C_5$alkoxy or nitro ; or

$R_1$ and $R_2$ together represent a radical $-O-CH_2-O-$ or $-O-CH_2-CH_2-O-$ ;

$R_5$ and $R_8$ independently of one another each represents hydrogen, halogen or methyl ;

$R_7$ and $R_8$ independently of one another each represents hydrogen, methyl or ethyl ;

X represents $-O-$ or $-S-$ ; and Y represents $-O-$, with the proviso that at least one of the substituents $R_1$ to $R_8$ is other than hydrogen when X and Y both represent $-O-$.

3. A compound according to claim 2 of formula Ia

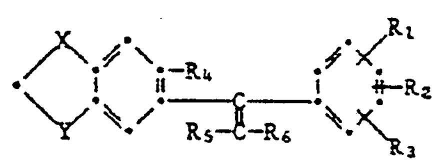

(Ia)

in which $R_1$ to $R_6$, X and Y have the meanings given in claim 2.

4. A compound according to claim 2 of formula Ib

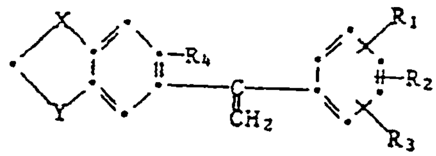

(Ib)

in which $R_1$ to $R_4$, X and Y have the meanings given in claim 2.

5. A compound according to any one of claims 1 to 4, characterised in that

$R_1$ and $R_4$ independently of one another each represents hydrogen, $C_1$-$C_5$alkoxy, $C_1$-$C_2$alkoxy substituted by from 1 to 5 fluorine or chlorine atoms, alkoxyalkoxy having a total of from 2 to 5 carbon atoms, or propynyloxy ;

$R_2$ and $R_3$ independently of one another each represents hydrogen, fluorine, chlorine, methoxy or ethoxy ; or

$R_1$ and $R_2$ together represent a radical —O-$CH_2$-O— or —O-$CH_2$-$CH_2$-O— ; and

$R_5$ and $R_6$ independently of one another each represents hydrogen, halogen or methyl, with the proviso that at least one of the substituents $R_1$ to $R_8$ is other than hydrogen when X and Y both represent —O—.

6. A compound of formula Ib according to claim 4, characterised in that
$R_1$ represents methoxy, ethoxy or trifluoromethoxy ;
$R_2$ and $R_3$ independently of one another each represents hydrogen or fluorine ; and
$R_4$ represents $C_1$-$C_8$alkoxy.

7. A compound according to any one of claims 1 to 6 of formula Ic

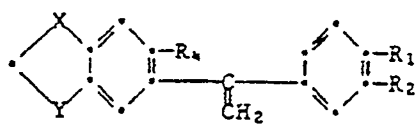

(Ic)

in which $R_1$, $R_2$ and $R_4$ and X and Y have the meanings given in claims 1 to 6 with the given proviso.

8. A compound according to any one of claims 1 to 7, characterised in that X and Y both represent —O— with the given proviso.

9. A compound according to any one of claims 1 to 7, characterised in that X represents —S— and Y represents —O—.

10. A compound according to any one of claims 1 to 9, characterised in that $R_2$ represents fluorine.

11. A compound according to claim 8 of formula

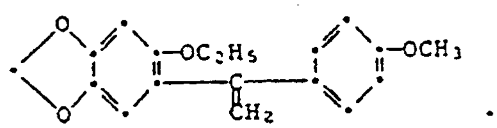

12. A compound according to claim 8 of formula

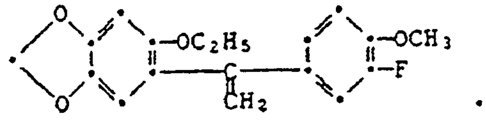

13. A compound according to claim 8 of formula

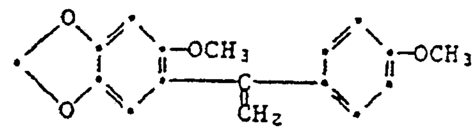

.

14. A compound according to claim 9 of formula

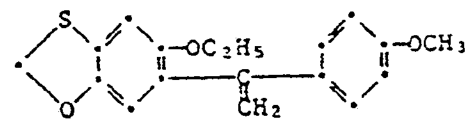

.

15. A compound according to claim 8 of formula

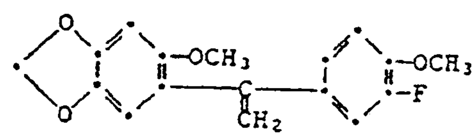

16. A compound according to claim 9 of formula

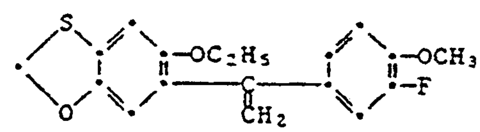

.

17. A compound according to claim 9 of formula

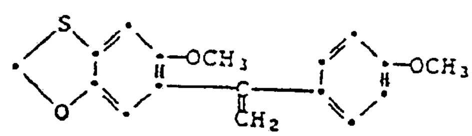

18. A compound according to claim 9 of formula

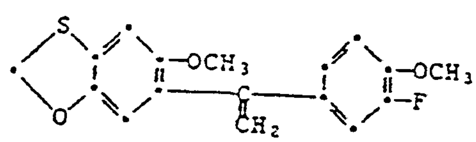

19. A process for the preparation of a compound of formula I according to any one of claims 1 to 18, characterised in that a compound of formula II

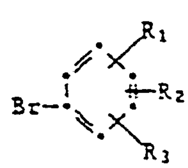

(II)

is reacted in a Grignard reaction in the presence of magnesium or under the action of a lithiumalkyl with a compound of formula III

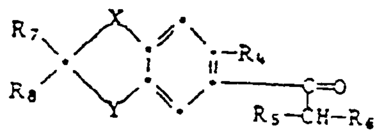

(III),

$R_1$ to $R_8$, X and Y in formulae II and III having the meanings given under claims 1 to 10.

20. A process for the preparation of a compound of formula I in which X and Y both represent —O—, according to any one of claims 1 to 8, 10 to 13 and 15, characterised in that a compound of formula IV

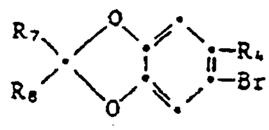

(IV)

is reacted in a Grignard reaction in the presence of magnesium or under the action of a lithiumalkyl with a compound of formula V

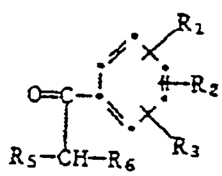

(V),

$R_1$ to $R_8$ in formulae IV and V having the meanings given under claims 1 to 7 and 10.

21. A compound of formula III according to claim 19 with the proviso that in those compounds of formula III in which $R_4$ represents hydrogen, hydroxy, $C_1$-$C_5$alkyl, $C_1$-$C_8$alkoxy, $C_1$-$C_3$alkoxy substituted by from 1 to 7 halogen atoms, alkoxyalkoxy having a total of from 2 to 6 carbon atoms, $C_3$-$C_5$alkenyloxy or $C_3$-$C_5$alkynyloxy,
$R_5$ and $R_6$ independently of one another each represents hydrogen, halogen or methyl,
$R_7$ and $R_8$ independently of one another each represents hydrogen, methyl or ethyl, and
X and Y independently of one another each represents —O— or —S—,
with the proviso that in those compounds of formula III in which

i) $R_4$, $R_7$ and $R_8$ represent hydrogen and X and Y both represent —O— and one of the two substituents $R_5$ and $R_6$ represents hydrogen, the other of those two substituents represents halogen ;

ii) $R_4$ represents hydroxy or methoxy, $R_7$ and $R_8$ represent hydrogen and X and Y both represent —O—, at least one of the two substituents $R_5$ and $R_6$ represents halogen or methyl ;

iii) $R_4$ represents hydrogen and of the two substituents $R_5$ and $R_6$ one represents hydrogen and the other represents chlorine and X represents —O— and Y represents —S—, of the substituents $R_7$ and $R_8$ either at least one represents hydrogen or one represents methyl and the other represents ethyl ;

iv) $R_4$, $R_5$ and $R_6$ represent hydrogen and X represents —O— and Y represents —S—, of the two substituents $R_7$ and $R_8$ one represents hydrogen and the other represents methyl or ethyl.

22. A process for the preparation of a compound of formula III according to claim 21, characterised in that a compound of formula VIII

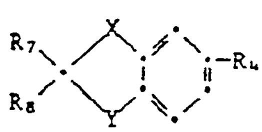

(VIII)

is reacted in the presence of a Friedel-Crafts catalyst with an acylhalide of formula IX

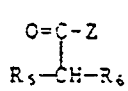

(IX)

in which $R_4$ to $R_8$, X and Y have the meanings given under claims 1 to 10 and Z represents a halogen atom, preferably chlorine.

23. A pesticidal composition comprising as active ingredient a compound of formula I according to any one of claims 1 to 18 together with suitable carriers and/or other adjuvants.

24. The use of a compound of formula I according to any one of claims 1 to 18 for controlling insects and representatives of the order Acarina in plants.

25. The use according to claim 24 for controlling larval stages of plant-destructive feeding insects.

26. The use according to claim 24 for controlling plant-destructive sucking insects.

27. A compound of formula I according to claims 1 to 18 for use in controlling ectoparasites in domestic animals and productive livestock.

28. A compound according to claim 27 for use in controlling ticks.

29. A method of controlling insects and representatives of the order Acarina, characterised in that these pests or their various stages of development or the locus thereof, with the exception of the human or animal body, are brought into contact with or treated with a pesticidally effective amount of a compound of formula I according to any one of claims 1 to 18 or with a composition comprising a pesticidally effective amount of this compound together with adjuvants and carriers.

## Revendications

1. Composé de formule I

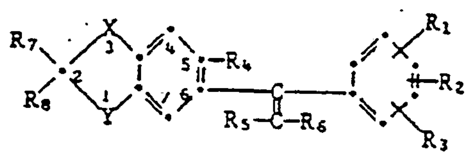

(I)

dans laquelle

$R_1$ et $R_4$ représentent indépendamment l'un de l'autre un hydrogène, hydroxy, alcoyle en $C_{1-5}$, alcoxy en $C_{1-8}$, alcoxy en $C_{1-3}$ substitué par de 1 à 7 atomes d'halogène, alcoxyalcoxy avec au total de 2 à 6 atomes de carbone, alcényloxy en $C_{3-5}$ ou alcynyloxy en $C_{3-5}$ ;

$R_2$ et $R_3$ représentent indépendamment l'un de l'autre un hydrogène, halogène, alcoyle en $C_{1-3}$, alcoxy en $C_{1-5}$ ou nitro ; ou

$R_1$ et $R_2$ représentent ensemble un radical —O-$CH_2$-O— ou —O-$CH_2$-$CH_2$-O— ;

$R_5$ et $R_6$ représentent indépendamment l'un de l'autre un hydrogène, halogène, ou méthyle ;

$R_7$ et $R_8$ représentent indépendamment l'un de l'autre un hydrogène, méthyle ou éthyle ; et

X et Y représentent indépendamment l'un de l'autre —O— ou —S—,

avec cette précision qu'au moins l'un des substituants $R_{1-8}$ a une signification différente d'hydrogène, lorsque X et Y représentent tous deux —O—.

2. Composé de formule I selon la revendication 1, caractérisé en ce que

$R_1$ et $R_4$ représentent indépendamment l'un de l'autre un hydrogène, alcoxy en $C_{1-8}$, alcoxy en $C_{1-3}$ substitué par de 1 à 7 atomes d'halogène, alcoxyalcoxy avec un total de 2 à 6 atomes de carbone, alcényloxy en $C_{3-5}$ ou alcynyloxy en $C_{3-5}$ ;

$R_2$ et $R_3$ représentent indépendamment l'un de l'autre un hydrogène, halogène, alcoyle en $C_{1-3}$, alcoxy en $C_{1-5}$ ou nitro ; ou

$R_1$ et $R_2$ représentent ensemble un radical —O-$CH_2$-O— ou —O-$CH_2$-$CH_2$-O— ;

$R_5$ et $R_5$ représentent indépendamment l'un de l'autre un hydrogène, halogène ou méthyle ;

$R_7$ et $R_8$ représentent indépendamment l'un de l'autre un hydrogène, méthyle ou éthyle ;

X représente —O— ou —S— ; et

Y représente —O—,

avec cette précision qu'au moins l'un des substituants $R_{1-8}$ a une signification différente d'hydrogène lorsque X et Y représentent tous deux —O—.

3. Composé selon la revendication 2, de formule Ia

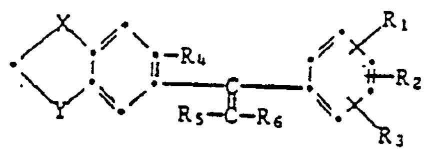

(Ia),

dans lequel $R_1$ à $R_6$, X et Y ont les significations données dans la revendication 2.

4. Composé selon la revendication 2, de formule Ib

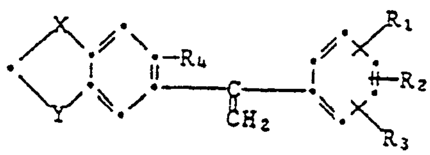

(Ib),

dans laquelle $R_1$ à $R_4$, X et Y ont les significations données dans la revendication 2.

5. Composés selon l'une des revendications 1 à 4, caractérisés en ce que

| | |
|---|---|
| $R_1$ et $R_4$ | représentent indépendamment l'un de l'autre un hydrogène, alcoxy en $C_{1-5}$, alcoxy en $C_{1-2}$ substitué par de 1 à 5 atomes de fluor ou de chlore, alcoxyalcoxy avec au total de 2 à 5 atomes de carbone ou propynyloxy ; |
| $R_2$ et $R_3$ | représentent indépendamment l'un de l'autre un hydrogène, fluor, chlore, méthoxy ou éthoxy ; ou |
| $R_1$ et $R_2$ | représentent ensemble un radical —O-$CH_2$-O— ou —O-$CH_2$-$CH_2$-O— ; et |
| $R_5$ et $R_6$ | représentent indépendamment l'un de l'autre un hydrogène, halogène ou méthyle, avec cette précision qu'au moins un des substituants $R_1$ à $R_8$ à une signification différente d'hydrogène lorsque X et Y représentent tous deux —O— |

6. Composés de formule Ib selon la revendication 4, caractérisés en ce que

| | |
|---|---|
| $R_1$ | représente un méthoxy, éthoxy ou trifluorométhoxy ; |
| $R_2$ et $R_3$ | représentent indépendamment l'un de l'autre un hydrogène ou un fluor ; et |
| $R_4$ | représente un alcoxy en $C_{1-8}$. |

7. Composés selon l'une des revendications 1 à 6, de formule Ic

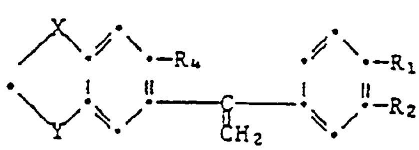

(Ic),

dans laquelle $R_1$, $R_2$ et $R_4$ ainsi que X et Y ont les significations données dans les revendications 1 à 6, en observant la précision donnée.

8. Composé selon l'une des,revendications 1 à 7, caractérisé en ce que X et Y représentent simultanément —O—, avec un maintien de la précision donnée.

9. Composé selon l'une des revendications 1 à 7, caractérisé en ce que X représente —S— et Y représente —O—.

10. Composé selon l'une des revendications 1 à 9, caractérisé en ce que $R_2$ représente un fluor.

11. Composé selon la revendication 8, de formule

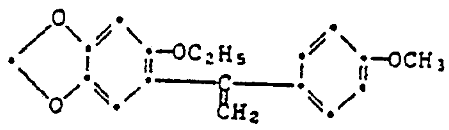

12. Composé selon la revendication 8, de formule

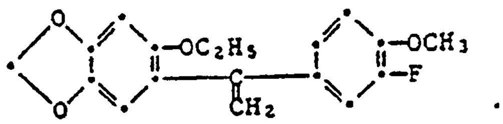

13. Composé selon la revendication 8, de formule

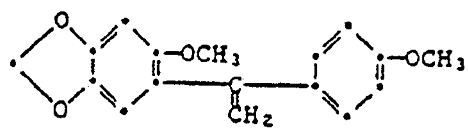

14. Composé selon la revendication 9, de formule

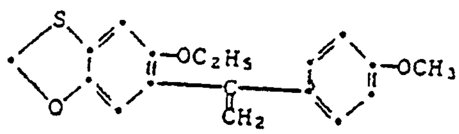

15. Composé selon la revendication 8, de formule

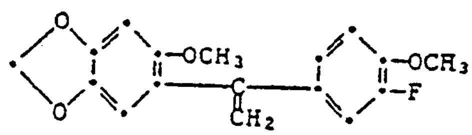

16. Composé selon la revendication 9, de formule

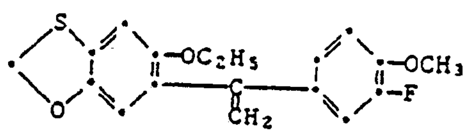

17. Composé selon la revendication 9, de formule

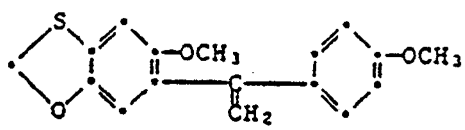

18. Composé selon la revendication 9, de formule

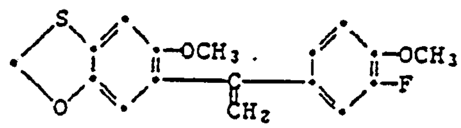

19. Procédé de préparation d'un composé de formule I selon l'une des revendications 1 à 18, caractérisé en ce qu'on fait réagir un composé de formule II

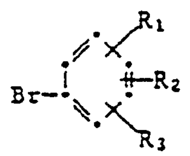

(II)

dans une réaction de Grignard en présence de magnésium ou sous l'action d'un alcoyl-lithium avec un composé de formule III

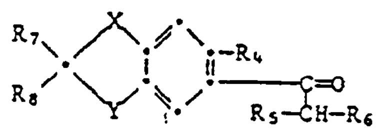

(III),

où, dans les formules II et III, $R_1$ à $R_8$, X et Y, ont les significations indiquées dans les revendications 1 à 10.

20. Procédé de préparation d'un composé de formule I, dans lequel X et Y représentent simultanément —O—, selon l'une des revendications 1 à 8, 10 à 13 et 15, caractérisé en ce qu'on fait réagir un composé de formule IV

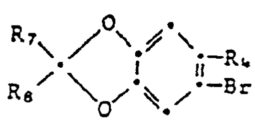

(IV)

dans une réaction de Grignard en présence de magnésium ou sous l'action d'un alcoyl-lithium avec un composé de formule V

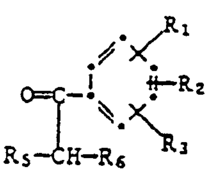

(V)

où dans les formules IV et V $R_1$ à $R_8$ ont les significations indiquées dans les revendications 1 à 7 et 10.

21. Composé de formule III selon la revendication 19, avec cette précision que dans les composés de formule II dans lesquels

| | |
|---|---|
| $R_4$ | représente un hydrogène, hydroxy, alcoyle en $C_{1-5}$, alcoxy en $C_{1-8}$, alcoxy en $C_{1-3}$ substitué par de 1 à 7 atomes d'halogène, alcoxyalcoxy avec au total de 2 à 6 atomes de carbone, alcényloxy en $C_{3-5}$ ou alcynyloxy en $C_{3-5}$, |
| $R_5$ et $R_6$ | représentent indépendamment l'un de l'autre un hydrogène, halogène ou méthyle, |
| $R_7$ et $R_8$ | représentent indépendamment l'un de l'autre un hydrogène, méthyle ou éthyle, et |
| X et Y | représentent indépendamment l'un de l'autre —O— ou —S— |

avec cette précision que dans les composés de formule III dans lesquels

i) $R_4$, $R_7$ et $R_8$ représentent un hydrogène et X et Y représentent tous deux —O— et l'un des substituants $R_5$ et $R_6$ représente un hydrogène, l'autre de ces deux substituants représente un halogène ;
ii) $R_4$ représente un hydroxy ou un méthoxy, $R_7$ et $R_8$ représentent un hydrogène ou X et Y représentent tous deux —O—, au moins l'un des deux substituants $R_5$ et $R_6$ représente un halogène ou un méthyle ;
iii) $R_4$ représente un hydrogène et parmi les deux substituants $R_5$ et $R_6$ l'un représente un hydrogène et l'autre un chlore et X représente —O— et Y représente —S—, parmi les substituants $R_7$ et $R_8$, au moins l'un représente un hydrogène ou l'un représente un méthyle et l'autre représente un éthyle ;
iv) $R_4$, $R_5$ et $R_6$ représentent un hydrogène et X représente —O— et Y représente —S—, parmi les deux substituants $R_7$ et $R_8$, l'un représente un hydrogène et l'autre représente un méthyle ou un éthyle.

22. Procédé de préparation d'un composé de formule III selon la revendication 21, caractérisé en ce qu'on fait réagir un composé de formule VIII

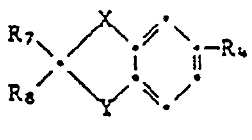

(VIII)

en présence d'un catalyseur de Friedel-Crafts avec un halogénure d'acyle de formule IX

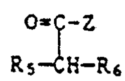

(IX)

où $R_4$ à $R_8$, X et Y ont les significations données dans les revendications 1 à 10 et Z représente un atome d'halogène, de préférence de chlore.

23. Agent de lutte antiparasitaire qui contient comme composant actif un composé de formule I selon l'une des revendications 1 à 18 avec des supports et/ou autres additifs appropriés.

24. Application d'un composé de formule I selon l'une des revendications 1 à 18, pour combattre les insectes et les représentants de l'Ordre des Acariens sur les plantes.

25. Application selon la revendication 24, pour combattre les stades larvaires des insectes phytophages.

26. Application selon la revendication 24 pour combattre les insectes suceurs qui causent des dommages aux plantes.

27. Composé de formule I selon les revendications 1 à 18, aux fins d'application pour combattre les ectoparasites chez les animaux domestiques et les animaux utiles.

28. Composé selon la revendication 27, aux fins d'application pour combattre les tiques.

29. Procédé de lutte contre les insectes et les représentants de l'Ordre des Acariens, caractérisé en ce qu'on met en contact ou traite ces parasites ou leurs différents stades de développement, ou leur habitat, à l'exception du corps humain ou animal, avec une quantité à action pesticide d'un composé de formule I selon l'une des revendications 1 à 18 ou avec un agent contenant, outre des additifs et supports, une quantité à action pesticide de ce composé.